(19) Europäisches Patentamt

European Patent Office

Office européen des brevets

(11) **EP 0 913 166 B1**

(12) **EUROPÄISCHE PATENTSCHRIFT**

(45) Veröffentlichungstag und Bekanntmachung des Hinweises auf die Patenterteilung:
**01.09.2004   Patentblatt 2004/36**

(51) Int Cl.$^7$: **A61N 1/37**

(21) Anmeldenummer: **98250385.6**

(22) Anmeldetag: **31.10.1998**

(54) **Elektrostimulator**

Electrostimulating device

Stimulateur électrique

(84) Benannte Vertragsstaaten:
**CH DE FR GB LI NL**

(30) Priorität: **31.10.1997  DE 19749710**

(43) Veröffentlichungstag der Anmeldung:
**06.05.1999   Patentblatt 1999/18**

(73) Patentinhaber: **BIOTRONIK Mess- und Therapiegeräte GmbH & Co Ingenieurbüro Berlin
12359 Berlin (DE)**

(72) Erfinder:
• **Busch, Ulrich, Dipl.-Ing.
10145 Berlin (DE)**

• **Bartels, Klaus, Dr. rer. nat.
10115 Berlin (DE)**

(74) Vertreter: **Eisenführ, Speiser & Partner
Patentanwälte Rechtsanwälte
Spreepalais am Dom
Anna-Louisa-Karsch-Strasse 2
10178 Berlin (DE)**

(56) Entgegenhaltungen:
**EP-A- 0 057 944          WO-A-98/19738
WO-A-99/58192          US-A- 4 245 643
US-A- 4 613 850          US-A- 5 436 566**

**Beschreibung**

Die Erfindung betrifft einen Elektrostimulator gemäß dem Oberbegriff des Anspruchs 1.

Es ist seit längerem bekannt, zur Behandlung von Herzfunktionsstörungen, insbesondere bei Bradykardiezuständen, implantierbare Herzschrittmacher zu verwenden, die über eine endokardial angeordnete Stimulationselektrode elektrische Stimulationsimpulse an das Herz abgeben, falls dieses selbst nicht oder nicht hinreichend schnell schlägt.

Da jeder Stimulationsimpuls zu einer partiellen Entladung der Herzschrittmacherbatterie führt, ist man zur Verlängerung der Batterielebensdauer bestrebt, die Amplitude der Stimulationsimpulse möglichst weit abzusenken, wobei jedoch zu beachten ist, daß das Herz bei einer Stimulation mit einer Amplitude unterhalb eines bestimmten Schwellwerts - auch als Reizschwellwert bezeichnet - nicht mehr mit einer Kontraktion reagiert.

Es ist deshalb ebenfalls bekannt, einen sogenannten Reizschwellwerttest durchzuführen, um den Reizschwellwert des Herzens individuell für jeden Herzschrittmacherträger zu ermitteln und die Amplitude der Stimulationsimpulse entsprechend programmieren zu können. Hierzu gibt der Herzschrittmacher nacheinander Stimulationsimpulse mit abnehmender Amplitude ab, wobei durch Auswertung eines extrakorporal abgenommenen Elektrokardiogramms (EKG) jeweils ermittelt wird, ob das Herz auf den vorangegangenen Stimulationsimpuls mit einer Kontraktion reagiert. Der Reizschwellwert des Herzens entspricht dann näherungsweise der Amplitude, bei der das Herz von dem Stimulationsimpuls gerade noch erregt wurde.

Problematisch ist hierbei jedoch, daß eine Änderung des Reizschwellwerts beispielsweise aufgrund von Änderungen der chronischen Reizschwelle im normalen Betrieb des Herzschrittmachers nicht erkannt wird, was entweder zu einer Stimulation mit unnötig hohen Amplituden oder - wesentlich schlimmer - zu einer erfolglosen Stimulation führen kann.

Es sind deshalb neuerdings Herzschrittmacher bekannt, die selbständig ermitteln, ob das Herz von einem Stimulationsimpuls erfolgreich stimuliert wird, und die Amplitude der Stimulationsimpulse entsprechend optimieren. Hierzu mißt der Herzschrittmacher über die Schrittmacherelektrode jeweils unmittelbar nach einem Stimulationsimpuls das sogenannte evozierte Potential, das die Herzmuskelkontraktion hervorruft und die Reizantwort auf den vorangegangenen Stimulationsimpuls darstellt. Problematisch ist hierbei, daß das Elektrodensystem, das zwei Metall-Elektrolyt-Grenzflächen einschließt, aufgrund seiner kapazitiven Eigenschaften durch einen Stimulationsimpuls elektrisch aufgeladen wird, so daß die evozierten Potentiale von den elektrischen Nachwirkungen eines Stimulationsimpulses (Artefakten auf beiden Grenzschichtkapazitäten) verdeckt werden können. Dieses Konzept eignet sich deshalb nur im Zusammenhang mit hochkapazitiven Elektroden, die aufgrund ihrer großen Kapazität von einem Stimulationsimpuls nur auf eine relativ geringe Spannung aufgeladen werden, die die Detektion des evozierten Potentials nicht stört.

Die Auswahl geeigneter Elektroden erfolgte bisher aufgrund einer extrakorporalen Messung der Elektrodenkapazität mittels separater Meßgeräte, was erhöhten Aufwand bei der Implantation bedeutet und den Nachteil hat, daß eine postoperative Änderung der Elektrodenkapazität vom Herzschrittmacher nicht erkannt wird. Probleme waren zudem bei Neu-Implantation eines Schrittmachers bei Weiterverwendung der bereits implantierten Elektrode zu erwarten. Aus der US-A-4 613 850 ist ein Elektrostimulator bekannt, welcher ausgebildet ist, mit einen Testpüls die Transferimpedanz und die Kapazität einer angeschlossenen Elektrode zu erfassen.

Der Erfindung liegt somit die Aufgabe zugrunde, einen Elektrostimulator zu schaffen, der die Messung der Elektrodenkapazität auch im implantierten Zustand ohne separate Geräte ermöglicht.

Die Aufgabe wird, ausgehend von einem Elektrostimulator gemäß dem Oberbegriff des Anspruchs 1, durch dessen kennzeichnende Merkmale gelöst.

Die Erfindung schließt die technische Lehre ein, zur Messung der Kapazität bzw. der komplexen Impedanz der Arbeitselektrode ein Meßgerät in die Stimulatoranordnung zu integrieren, das bei der Abgabe eines Impulses die Spannung und den Strom am Ausgangsanschluß als der Schnittstelle zwischen Stimulator und Arbeits- bzw. Stimulationselektrode mißt, und mittels einer Auswertungseinrichtung aus den gemessenen Werten ein die Elektrodenkapazität widerspiegelndes Ausgangssignal zu berechnen.

Die Erfindung ist nicht auf in-vivo-Anwendungen begrenzt, sondern bezieht sich vielmehr auch auf den in-vitro-Bereich unter Verwendung von Elektrolytlösungen.

In einer Variante der Erfindung erzeugt der Impulsgenerator zur Bestimmung der Elektrodenkapazität einen Impuls mit einer vorgegebenen elektrischen Ladung $Q$, beispielsweise einen Konstantstromimpuls mit vorgegebener Amplitude und Dauer: Anschließend mißt das Meßgerät am Ausgangsanschluß zwischen Herzschrittmacher und Stimulationselektrode, auf welche Spannung $U$ die Stimulationselektrode durch den Impuls aufgeladen wurde und gibt diesen Meßwert an eine nachgeschaltete Recheneinheit weiter, die daraus nach der Formel

$$\frac{1}{C_{EI}} + \frac{1}{C_{CASE}} = \frac{U}{Q}$$

bei bekannter Gehäusekapazität $C_{CASE}$ die Elektrodenkapazität $C_{EL}$ berechnet. Die Erfindung ist in dieser Variante jedoch nicht auf einen Konstantstromimpuls beschränkt. Entscheidend ist, daß die mit dem Impuls abgegebene elektrische Ladung *Q* bzw. der während der Impulsdauer fließende Strom bekannt ist. Hierzu ist es wahlweise möglich, einen Impuls mit vorgegebener Ladung zu erzeugen oder während der Abgabe eines Impulses mit bekanntem Stromverlauf die Zeit zu messen. Vorzugsweise wird ein Konstantstromimpuls verwendet.

Zur Verbesserung der Meßgenauigkeit der Kapazitätsmessung infolge einer Verringerung von Polarisationseffekten am Elektrodensystem kann der Konstantstromimpuls als Doppelimpuls mit zueinander inverser Stromrichtung der beiden Teilimpulse ausgeführt werden.

In einer anderen Variante der Erfindung ist vorgesehen, zur Bestimmung der Elektrodenkapazität einen Impuls mit vorgegebenem Spannungsverlauf, vorzugsweise einen Konstantspannungsimpuls, abzugeben.

Betrachtet man die Stimulationselektrode elektrisch als Reihenschaltung aus einer Kapazität $C_{El}$ und einem ohmschen Widerstand $R_{El}$, so nimmt die Spannung über der Elektrodenkapazität bei einem Konstantspannungsimpuls während der Impulsdauer exponentiell zu und nähert sich asymptotisch der Spannungsamplitude $U_{Stim}$ des Impulses. Die Elektrodenkapazität $C_{El}$ ergibt sich dann nach der Formel

$$ C_{El} = \frac{-T}{R \cdot \ln\left(1 - \frac{U_{E1}}{U_{Stim}}\right)} $$

aus der Impulsdauer *T*, der Spannungsamplitude $U_{Stim}$ des Impulses, der am Ausgangsanschluß nach dem Impulsende gemessenen Elektrodenspannung $U_{El}$ sowie dem ohmschen Ladewiderstand *R*, der sich aus dem ohmschen Widerstand $R_{EL}$ der Elektrode und im Ladekreis vorhandenen weiteren ohmschen Widerständen zusammensetzt, die als bekannt vorausgesetzt werden.

Gemäß einer anderen Variante der Erfindung ist die Schrittmacherelektrode Bestandteil eines Schwingkreises, wobei die Elektrodenkapazität aufgrund ihres Einflusses auf das Schwingungsverhalten des Schwingkreises bestimmt werden kann. Der Herzschrittmacher weist in dieser Variante intern eine Induktivität auf, die mit dem Ausgangsanschluß verbunden oder durch ein Schaltelement verbindbar ist. Die Induktivität kann hierbei mit der Elektrodenkapazität wahlweise in Reihe oder parallel geschaltet werden. Die Anregung des hierdurch gebildeten Schwingkreises erfolgt durch einen Schwingungsgenerator, der ebenfalls wahlweise mit der Schnittstelle verbunden oder mit dieser durch ein Schaltelement verbindbar ist.

In einer Ausführungsform dieser Variante erzeugt der Schwingungsgenerator ein vorzugsweise sinusförmiges Schwingungssignal konstanter Frequenz und Spannungsamplitude, so daß der durch den Schwingkreis fließende Strom von der Frequenzabstimmung zwischen Schwingungsgenerator einerseits und Schwingkreis andererseits abhängt. Die Messung des über die Schnittstelle fließenden Stroms ermöglicht dann die Berechnung der Elektrodenkapazität aus der Frequenz des Schwingungsgenerators und der Induktivität des Schwingkreises.

In einer anderen Ausführungsform dieser Variante ist dagegen vorgesehen, durch eine Änderung der Frequenz des Schwingungsgenerators bei gleichzeitiger Messung des Stroms die Resonanzfrequenz des die Phasengrenzkapazität enthaltenden Schwingkreises zu ermitteln, was dann in einfacher Weise gemäß der Thomson schen Schwingungsformel die Berechnung der Elektrodenkapazität ermöglicht.

Eine weitere Variante ist die Messung eines Impedanzspektrogramms des Elektrodensystems mit einem kontinuierlichen Schwingungsgenerator. Dabei wird durch Einprägung eines frequenzvariablen Konstantstrom- oder Konstantspannungssignals und Messung von Spannung bzw. Strom an dem Elektrodensystem der Verlauf der Elektrodenimpedanz in Abhängigkeit von der Signalfrequenz aufgenommen. Aus dem Spektrum können die Helmholtzkapazität und darüber hinaus Elektrolyt- und Faradaywiderstand kalkuliert werden.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1      als bevorzugtes Ausführungsbeispiel der Erfindung einen Herzschrittmacher mit einer Stimulationselektrode als Funktion-Blockschaltbild,

Figur 2      den Impulsgenerator des in Figur 1 dargestellten Herzschrittmachers als vereinfachtes Schaltbild,

Figur 3a und 3b      den Spannungsverlauf an der Schnittstelle bzw. an den verschiedenen Bauelementen im Ausgangsstromkreis und

Figur 4      als weiteres Ausführungsbeispiel ein FunktionsBlockschaltbild eines Herzschrittmachers.

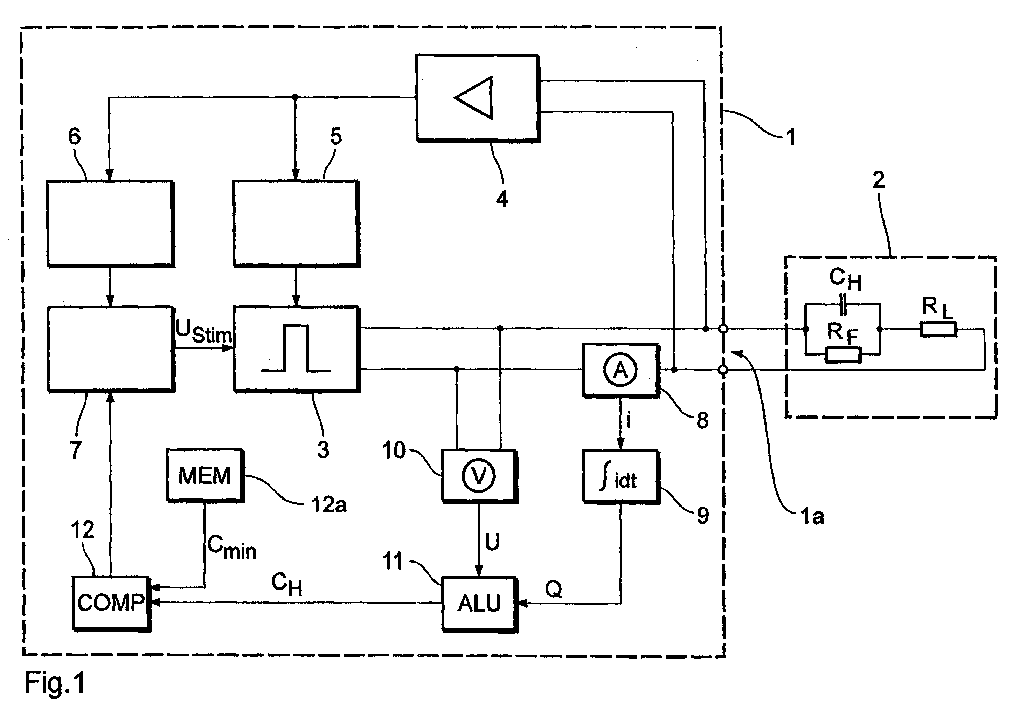

Der in Figur 1 als Blockschaltbild dargestellte Herzschrittmacher 1 ermöglicht über eine - hier im Ersatzschaltbild dargestellte - Stimulationselektrode 2 die Abgabe von Stimulationsimpulsen an das Herz. Zur Vereinfachung ist hier vom Elektrodensystem nur eine der Elektrode-Elektrolyt-Grenzflächen im Ersatzschaltbild gezeigt.

Das Ersatzschaltbild der endokardial anzuordnenden Schrittmacherelektrode 2 soll die wesentlichen elektrischen Eigenschaften der Metall/Gewebe-Grenzschicht an der Schrittmacherelektrode wiedergeben. So bildet sich an der Grenzschicht zwischen der Schrittmacherelektrode 2 und dem umliegenden (nicht dargestellten) Gewebe eine sogenannte Helmholtz-Doppelschicht aus, die durch eine Parallelschaltung eines Kondensators $C_H$ und eines ohmschen Widerstandes $R_F$ nachgebildet wird, wobei zu beachten ist, daß der ohmsche Widerstand $R_F$ der Helmholtz-Doppelschicht stark vom Elektrodenpotential abhängig ist. Weiterhin weist die Schrittmacherelektrode 2 einen ohmschen Leitungswiderstand $R_L$, der mit der vorstehend beschriebenen Impedanz der Helmholtz-Doppelschicht in Reihe geschaltet ist, sowie - genau genommen - einen (bei kleinen Elektrodenflächen wichtigen) dazu in Reihe liegenden Elektrolytwiderstand auf, der allerdings hier nicht gesondert dargestellt ist.

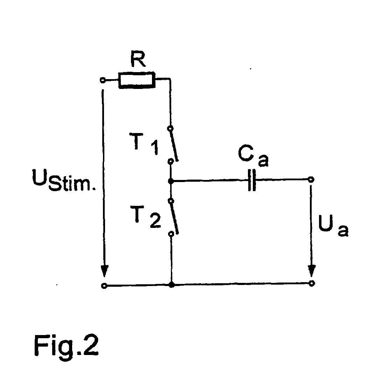

Zum einen ermöglicht die Schrittmacherelektrode 2 die Abgabe von Stimulationsimpulsen an das Herz. Die Erzeugung der Stimulationsimpulse erfolgt hierbei in herkömmlicher Weise durch den in Figur 2 detaillierter dargestellten Impulsgenerator 3, der ausgangsseitig zum Anschluß der Schrittmacherelektrode 2 einen Ausgangsanschluß 1a aufweist.

Zum anderen dient die Schrittmacherelektrode 2 zur Detektion der von spontanen, nicht stimulierten Herzaktionen herrührenden elektrischen Aktivität des Herzens, um die Abgabe eines Stimulationsimpulses gegebenenfalls inhibieren zu können. Zum einen wird hierdurch der Eigenrhythmus des Herzens weitestgehend aufrechterhalten. Zum anderen wird so eine unnötige Stimulation vermieden, was zu einer Verlängerung der Batterielebensdauer des Herzschrittmachers 1 beiträgt. Hierzu weist der Herzschrittmacher 1 einen Eingangsverstärker 4 auf, der die über die Schrittmacherelektrode 2 aufgenommenen elektrischen Herzsignale verstärkt und zur Detektion von spontanen Herzaktionen einem Signaldetektor 5 zuführt. Erkennt der Signaldetektor 5 eine spontane Herzaktion, so gibt er ein Inhibierungssignal an den Impulsgenerator 3, der daraufhin seine internen Zeitgeber zurücksetzt und die Abgabe eines Stimulationsimpulses aussetzt.

Darüber hinaus ermöglicht die Stimulationselektrode 2 auch die Detektion der Reizantwort des Herzens, was letztlich eine Anpassung der Stärke der Stimulationsimpulse an den individuellen Reizschwellwert des Herzschrittmacherträgers ermöglicht.

Eine erfolgreiche Stimulation des Herzens setzt - vereinfacht gesagt - voraus, daß die Amplitudenzeitfläche der einzelnen Stimulationsimpulse den individuellen Reizschwellwert überschreitet, damit das Herz auf einen Stimulationsimpuls mit einer Kontraktion reagiert. (Streng genommen, ist die dem Fachmann bekannte Chronaxie-Rheobase-Beziehung zu beachten.)

Die Einstellung der Stärke der Stimulationsimpulse erfolgt durch eine Steuerschaltung 7, die eingangsseitig mit dem Signaldetektor 6 verbunden ist und die Stimulationsspannung $U_{Stim}$ langsam absenkt, solange das Herz erregt wird und die Stimulationsspannung stufenartig anhebt, falls im Anschluß an einen Stimulationsimpuls keine Kontraktion des Herzmuskels detektiert wird.

Einerseits ist es wünschenswert, die Amplitudenzeitfläche der Stimulationsimpulse möglichst weit abzusenken, um im Interesse einer Verlängerung der Batterielebensdauer Energie zu sparen. Andererseits muß sichergestellt sein, daß die Stimulationsimpulse hinreichend stark sind, um das Herz zu erregen. Der Herzschrittmacher 1 überprüft deshalb jeweils nach einem Stimulationsimpuls die über die Schrittmacherelektrode 2 aufgenommenen elektrischen Herzsignale und ermittelt, ob evozierte Potentiale auftreten, die die Reizantwort auf den unmittelbar vorangegangenen Stimulationsimpuls darstellen. Das Ausgangssignal des Eingangsverstärkers 4 wird deshalb zur Detektion evozierter Potentiale einem speziellen Signaldetektor 6 zugeführt.

Zur Erzeugung eines Stimulationsimpulses wird der in Figur 2 dargestellte Transistor $T_1$ durch eine hier nicht dargestellte Steuerelektronik geschlossen, während Transistor $T_2$ geöffnet ist. Da der Ausgangskondensator $C_a$ zu Beginn eines Stimulationsimpulses vollständig entladen ist, liegt an der Schnittstelle zunächst die volle Spannung $U_{Stim}$ an. Während der Dauer des Stimulationsimpulses wird der Ausgangskondensator $C_a$ jedoch durch den über die Schnittstelle fließenden Strom aufgeladen, was zu einer exponentiellen Abnahme der an der Schnittstelle anliegenden Spannung jeweils während eines Impulses führt. Der Ausgangskondensator $C_a$ begrenzt so die während eines Stimulationsimpulses maximal abfließende Ladung auf den Wert, der - unter vereinfachender Annahme einer rein ohmschen Last - zur Aufladung des Ausgangskondensators $C_a$ auf die volle Spannung $U_{Stim}$ erforderlich ist.

Unmittelbar nach jedem Stimulationsimpuls sind also sowohl der Ausgangskondensator $C_a$ als auch die Helmholtz-Kapazität $C_H$ des Elektrodenpaares aufgeladen.

Zum einen wird durch die Spannung des Ausgangskondensators $C_a$ die beim nächsten Stimulationsimpulse maximal erreichbare Stimulationsspannung verringert, so daß unmittelbar nach einem Stimulationsimpuls kein neuer Impuls mit der vollen Spannung erzeugt werden kann.

Zum anderen stört die Aufladung der Elektrodenkapazität $C_H$ die Messung der natürlichen Herzaktivität, da die elektrischen Herzsignale von der Spannung der Elektrodenkapazität $C_H$ überlagert werden.

Nach dem Ende eines Stimulationsimpulses wird deshalb der Transistor $T_2$ geschlossen, während der Transistor $T_1$ geöffnet ist, so daß sich der Ausgangskondensator $C_a$ und die Elektrodenkapazität $C_H$ relativ rasch entladen. Der Entladevorgang verläuft hierbei hinreichend schnell, um nach einem Stimulationsimpuls die nächste natürliche, nicht stimulierte Herzaktion detektieren zu können, was relativ einfach ist, da das Herz während der auf eine Stimulation folgenden Refraktärzeit ohnehin keine spontane Eigenaktivität zeigt.

Die Detektion der Reizantwort des Herzens ist dagegen wesentlich schwieriger, da die evozierten Potentiale in sehr geringem zeitlichen Abstand zu dem Stimulationsimpuls auftreten. Die Messung der Reizantwort ist deshalb nur bei hochkapazitiven Elektroden möglich, die durch einen Stimulationsimpuls aufgrund ihrer großen Kapazität nur auf eine relativ geringe Spannung aufgeladen werden, die nach dem Autoshort die Messung der evozierten Potentiale nicht stört.

Die vorstehend beschriebene automatische Optimierung der Amplitude der Stimulationsimpulse setzt deshalb eine hinreichend große Kapazität der Schrittmacherelektrode 2 voraus. Bei zu niedriger Elektrodenkapazität sollte diese Funktion hingegen inaktiv geschaltet sein.

Der Herzschrittmacher 1 ermittelt deshalb die Kapazität des Elektrodensystems (und somit indirekt der Schrittmacherelektrode 2) und schaltet die automatische Optimierung der Impulsstärke aus, falls die Kapazität einen vorgegebenen Minimalwert unterschreitet.

Hierzu weist der Herzschrittmacher 1 ein Strommeßgerät 8 auf, das im Ausgangsstromkreis angeordnet ist und bei jedem Stimulationsimpuls bzw. speziellen Meßimpuls den abfließenden Strom mißt. Das Ausgangssignal des Strommeßgeräts 8 wird nachfolgend einem Integrator 9 zugeführt, der aus dem Stromverlauf die während eines Stimulationsimpulses abfließende elektrische Ladung ermittelt. Weiterhin ist ein Spannungsmeßgerät 10 vorgesehen, das unmittelbar nach dem Ende eines Stimulationsimpulses bzw. nach oder während eines Meßimpulses die Spannung an der Schnittstelle mißt, die im wesentlichen gleich der Ladespannung der Elektrodenkapazität ist. Im Falle einer Konstantstrommessung (siehe dazu weiter unten) ist ein Strommeßgerät nicht erforderlich.

Mit den Ausgängen des Integrators 9 und des Spannungsmessers 11 ist eingangsseitig eine Verarbeitungseinheit 11 (ALU - <u>a</u>rithmetical <u>l</u>ogical <u>u</u>nit) verbunden, die aus der über der Elektrodenkapazität gemessenen Spannung $U$ und der abgeflossenen elektrischen Ladung die Elektrodenkapazität $C_H$ nach der Formel

$$C_H = \frac{Q}{U}$$

berechnet.

Die auf diese Weise ermittelte Elektrodenkapazität $C_H$ wird einem Eingang einer Vergleichereinheit 12 zugeführt, in der sie mit einem am anderen Eingang anliegenden Minimalwert $C_{Min}$ verglichen wird, der zur Detektion der evozierten Potentiale und damit zur Durchführung der automatischen Optimierung der Impulsamplitude erforderlich und in einem programmierbaren Vergleichswertspeicher 12a gespeichert ist.

Liegt die gemessene Elektrodenkapazität oberhalb dieses Minimalwerts, so wird über den Ausgang der Vergleichereinheit an einen Eingang der Steuerschaltung 7 ein entsprechendes Signal ausgegeben, woraufhin die Steuerschaltung 7 die Stimulationsimpulsamplitude in der weiter oben beschriebenen Weise optimiert.

Unterschreitet die Elektrodenkapazität $C_H$ dagegen den erforderlichen Minimalwert $C_{Min}$, so wird die Optimierung der Impulsamplitude gesperrt, und die Steuerschaltung 7 setzt die Stimulationsamplitude auf einen voreingestellten Wert, der eine sichere Stimulation des Herzens gewährleistet.

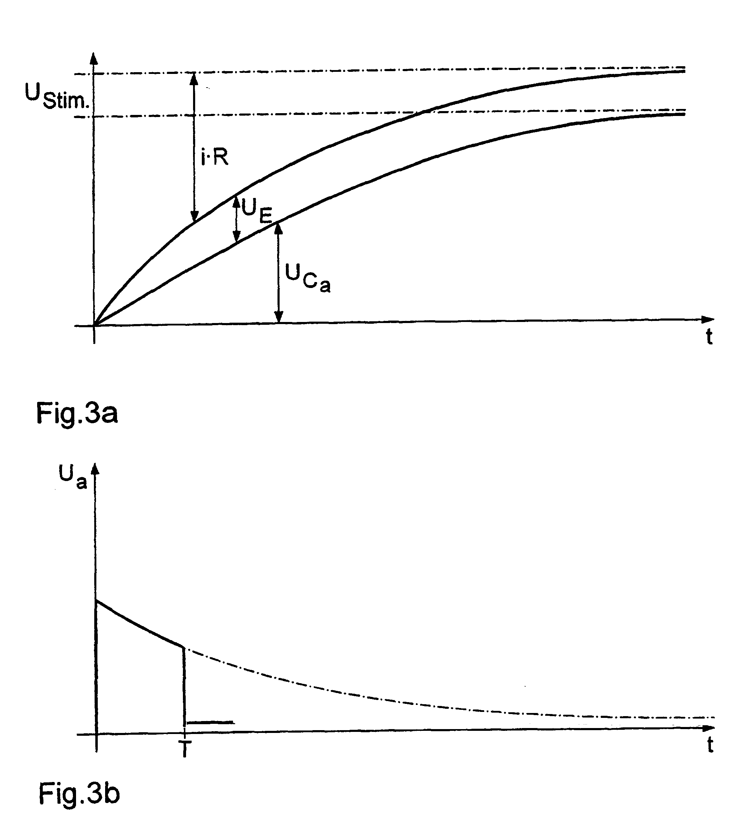

Der Spannungsverlauf an den verschiedenen Bauelementen im Ausgangsstromkreis ist detailliert in Figur 3a dargestellt, wobei angenommen wird, daß vor dem Ausgangskondensator ein Konstantspannungsimpuls mit der Amplitude $U_{Stim}$ erzeugt wird. Da sowohl Ausgangskondensator als auch Elektrodenkapazität zu Beginn des Stimulationsimpulses vollständig entladen sind, fällt die gesamte Stimulationsspannung zunächst über den im Ausgangsstromkreis angeordneten ohmschen Widerständen ab. Während des Stimulationsimpulses werden die beiden Kapazitäten jedoch aufgeladen, so daß der Strom exponentiell abfällt.

Figur 3b zeigt den am Ausgangsanschluß für die Schrittmacherelektrode meßbaren Spannungsverlauf. In Übereinstimmung mit Figur 3a entspricht die Ausgangsspannung zu Beginn des Stimulationsimpulses der gesamten Spannung $U_{Stim}$, da der Ausgangskondensator zunächst leer ist. Während des Stimulationsimpulses wird der Ausgangskondensator jedoch aufgeladen, was zu einem exponentiellen Abfall der Ausgangsspannung bis zum Ende des Stimulationsimpulse zum Zeitpunkt t=T führt. Zu diesem Zeitpunkt geht der Strom im Ausgangsstromkreis sprungartig auf Null zurück, so daß die Ausgangsspannung auf die Spannung über der Elektrodenkapazität abfällt, was unmittelbar nach dem Ende des Stimulationsimpulses die Bestimmung der Elektrodenkapazität durch eine einfache Spannungsmessung ermöglicht.

Lineare Beziehungen bestehen hierbei bis zu Artefakt-Spannungswerten von etwa 0,5V; der Aufbau höherer Spannungen sollte daher im Interesse der Aussagefähigkeit der Messungen vermieden werden. Aus diesem Grunde ist auch der unmittelbare Einsatz üblicher Stimulationsimpulse mit einer Amplitude von einigen Volt als Meßimpulse nur für Elektroden mit recht hoher Kapazität sinnvoll - besser sollte auf niedrigere Amplitudenwerte zurückgegriffen werden, die bei modernen Schrittmacherschaltungen ebenfalls programmierbar sind.

Zudem sollte die Spannungsmessung bevorzugt nicht sofort nach Beendigung des Meßimpulses erfolgen, sondern erst nach einer vorbestimmten Abklingzeit von einigen Millisekunden. Schließlich ist anzumerken, daß sowohl bei einer Potentialpuls- als auch bei einer Konstantstrommessung streng genommen an der Elektrode nicht nur die Spannung nach dem Impuls, sondern die Spannungsdifferenz nach und vor dem Impuls gemessen werden muß, da dem eigentlichen Artefakt eine (ohne vorherige Messung unbekannte) Offsetspannung überlagert ist. Entsprechend wäre Fig. 1 um einen Meßwertspeicher und eine Subtraktionsstufe - dem Spannungsmeßgerät 10 nach- und der ALU 11 vorgeschaltet - zu ergänzen.

Die oben beschriebene Meßtechnik kann in vorteilhafter Weise dahingehend modifiziert werden, daß ein Konstantstrom angewandt wird. Hierbei wird zur Konstanthaltung des durch die Meßelektrode fließenden Stromes ein galvanostatischer Regelkreis gebildet, der - in an sich bekannter Weise - eine stromzuführende Gegenelektrode und grundsätzlich zudem eine stromfreie Referenzelektrode sowie einen Meßstrom- und einen Leistungsverstärker einschließt. Die Messung des an der zu messenden Elektrode anliegenden Potentials erfolgt als nahezu belastungslose Abtastung mittels hochohmiger Sonde.

In praxi kann bei der Prüfung von implantierbaren Schrittmacherelektroden im Hinblick auf die sehr kleinen Polarisationsströme auf eine Referenzelektrode verzichtet und mit hinreichender Genauigkeit für bipolare Systeme zwischen Spitze und Ring und für unipolare Systeme zwischen Spitze und Schrittmachergehäuse gemessen werden.

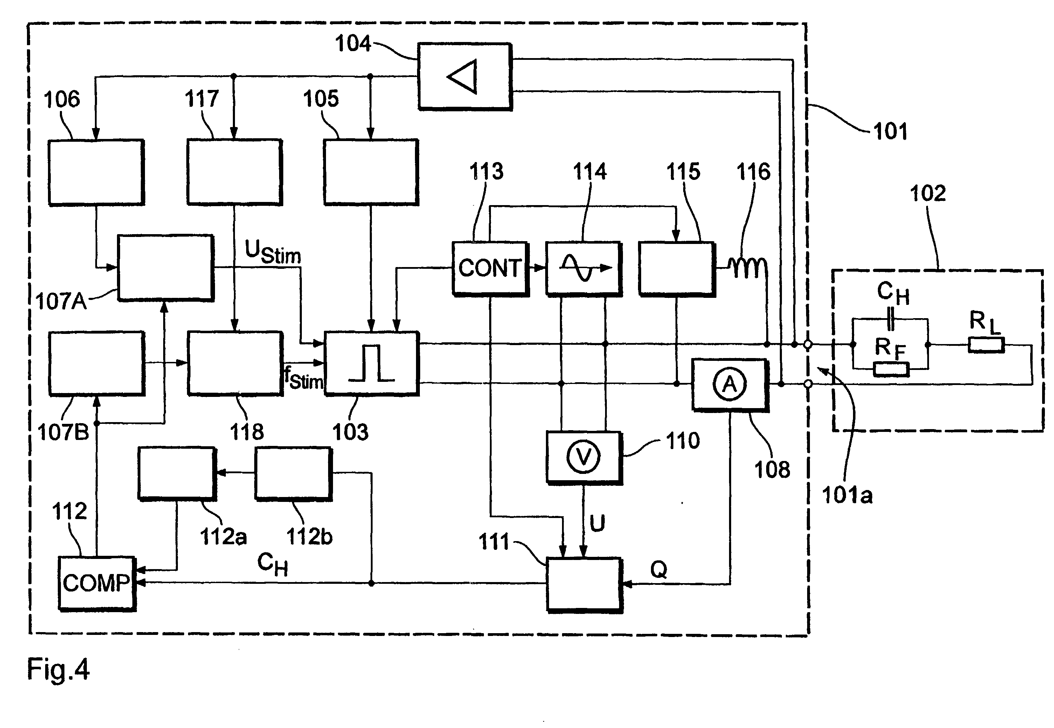

In Fig. 4 ist, wieder in Form eines Funktions-Blockschaltbildes, eine gegenüber Fig. 1 modifizierte Ausführung eines Herzschrittmachers dargestellt. Prinzipiell übereinstimmende Funktionskomponenten sind mit an Fig. 1 angelehnten Bezugsziffern (etwa für die Elektrode Ziffer 102 als funktionell im wesentlichen übereinstimmend mit Ziffer 2) bezeichnet und werden nachfolgend nicht nochmals erläutert.

Der in Fig. 4 skizzierte Schrittmacher unterscheidet sich von dem in Fig. 1 dargestellten hauptsächlich durch Mittel zur Adaption der Stimulationsrate und eine veränderte Meßund Auswertungsanordnung zur Prüfung der Elektrodenkapazität.

Die (an sich bekannten) Mittel zur Ratenadaption umfassen einen dem Ausgang des Eingangsverstärkers 4 nachgeschalteten QT-Intervall-Detektor 117 und eine mit dessen Ausgang verbundene Ratenadaptionsschaltung 118. Im QT-Intervall-Detektor 117 wird der Zeitabstand zwischen einem Stimulationsimpuls und einem vorbestimmten Abschnitt des evozierten Herzsignals (T-Welle) festgestellt, und in der Ratenadaptionsschaltung 118 wird aufgrund des gemessenen Zeitabstandes und anhand einer vorgespeicherten Wertetabelle ein Ratensteuersignal erzeugt, das schließlich dem Stimulationsimpulsgenerator 103 zugeführt wird.

Ebenso wie auf die oben beschriebene Weise die automatische Einstellung der Stimulationsamplitude (hier über eine erste Steuerschaltung 107A) inhibiert werden kann, so kann die Funktion der Ratenadaptionsschaltung 118 über eine ausgangsseitig mit der Ratenadaptionsschaltung verbundene zweite Steuerschaltung 107B inhibiert werden, falls die Elektrodenkapazität zu gering ist und deshalb die Gefahr besteht, daß die über die Elektrode 102 erfaßten evozierten Potentiale verfälscht sind.

Die Prüfung der Elektrodenkapazität erfolgt bei der Anordnung nach Fig. 4 - gesteuert durch eine Prüfsteuerschaltung (Controller) 113 - in Pausen zwischen den Stimulationimpulsen mittels eines abstimmbaren Wechselstromgenerators 114. Nach einem im Programmspeicher der Prüfsteuerschaltung 113 gespeicherten Programmablauf wird die Frequenz des Wechselstromgenerators 114 (bevorzugt innerhalb eines Bereiches zwischen 0,1 Hz und 10 kHz) schrittweise verändert und jeweils bei jedem Schritt die zur Elektrode 102 führende Ausgangleitung für eine vorbestimmte Zeitspanne über einen Schalter 115 mit einer bekannten Induktivität 116 verbunden. Dadurch wird als Last für den Wechselstromgenerator 114 jeweils ein Schwingkreis aus der Induktivltät 115 und der Elektrodenkapazität $C_H$ gebildet. Eine Spannungs- und Strommessung mittels des Strommeßgerätes 108 und des Spannungsmeßgerätes 110 in jedem Schritt ermöglicht die Feststellung einer Resonanzfrequenz des Schwingkreises und damit der Kapazität $C_H$ in einer Auswertungseinheit 111.

Ein letztes besonderes Merkmal der Anordnung nach Fig. 4 besteht im Vorsehen einer Bezugswert-Anpassungsstufe 112b, die einen als gleitender Mittelwert der über einen vorbestimmten Zeitraum gemessenen Elektrodenkapazitäten bestimmten Minimalwert an den Vergleichswertspeicher 112a liefert. Hierdurch kann erreicht werden, daß Langzeitschwankungen der Elektrodenkapazität ohne Einfluß auf die Steuer- bzw. Inhibierungsfunktionen der Steuerschaltungen 107A, 107B bleiben.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei anders gearteten Ausführungen Gebrauch machen.

So kann in einer weiteren Abwandlung des Meßprinzips die Elektrodenkapazität auch über eine Zeitmessung festgestellt werden, und zwar durch Erfassung des Zeitpunktes nach Anlegung eines Konstantstromimpulses an die Elektrode, zu dem auf der Elektrode eine vorgegebene Spannung auftritt, etwa eine Spannung von 300 mV.

Die Abschaltung (Inhibierung) der Verarbeitung von über die Schrittmacherelektrode aufgenommenen und u.U. verfälschten Signalen bei zu niedriger Elektrodenkapazität bzw. von auf dieser Auswertung basierten Funktionen ist nicht nur im Zusammenhang mit der automatischen Einstellung der Stimulationsamplitude oder der Ratenadaption - wie oben beschrieben - sondern auch in anderen Zusammenhängen denkbar.

**Patentansprüche**

1.  Elektrostimulator (1; 101) mit

    einem Ausgangsanschluß (1a; 101a) für eine Arbeitselektrode (2; 102) sowie

    einem ausgangsseitig mit dem Ausgangsanschluß verbundenen Prüfsignalgenerator (3; 114) zur Erzeugung eines impulsförmigen oder periodisch veränderlichen Prüfsignals und Zuführung zum Ausgangsanschluß,

    einer eingangssseitig mit dem Ausgangsanschluß verbundenen parallel zu dem Prüfsignalgenerator (3; 114) geschalteten ersten Meßeinrichtung (10; 110) zur Messung der am Ausgangsanschluß anliegenden elektrischen Spannung,

    sowie
    eine eingangsseitig mindestens mittelbar mit der ersten Meßeinrichtung verbundene Auswertungseinrichtung (11; 111) zur Erzeugung eines die Kapazität der Arbeitselektrode widerspiegelnden Ausgangssignals in Abhängigkeit von Strom und/oder Spannung am Ausgangsanschluß,
    **dadurch gekennzeichnet, daß**
    zur Messung des bei Ausgabe eines Prüfsignals über den Ausgangsanschluß (1a; 101a) fließenden Stromes als zweite Meßeinrichtung ein mit der Auswertungseinrichtung (11; 111) verbundenes, mit dem Prüfsignalgenerator in Reihe geschaltetes Strommeßgerät (8; 108)vorgesehen ist.

2.  Elektrostimulator nach Anspruche 1, **dadurch gekennzeichnet, daß** dem Strommeßgerät (8) zur Bestimmung der bei dem Impuls abfließenden elektrischen Ladung ein ausgangsseitig mit der Auswertungseinrichtung (11) verbundener Integrator (9) nachgeschaltet ist.

3.  Elektrostimulator nach Anspruch 1 , **dadurch gekennzeichnet, daß** der Prüfsignalgenerator (3; 114) zur Erzeugung elektrischer Impulse mit einer wesentlich unterhalb der Amplitude eines Stimulationsimpulses liegenden Amplitude ausgebildet ist.

4.  Elektrostimulator nach Anspruch 1 oder 4, **dadurch gekennzeichnet, daß**

    die erste Meßeinrichtung ein parallel zu dem Prüfsignalgenerator (3) geschaltetes Spannungsmeßgerät (10; 110) aufweist und

    dem Prüfsignalgenerator (3) zur Konstanthaltung des durch die Meßelektrode fließenden Stromes ein galvanostatischer Regelkreis zugeordnet ist, der eine stromzuführende Gegenelektrode sowie einen Meßstrom- und einen Leistungsverstärker aufweist.

5.  Elektrostimulator nach Anspruch 4, **dadurch gekennzeichnet, daß**

    der durch die Meßelektrode fließende Konstantstrom pulsförmig ist und

    daß unmittelbar vor und nach Beginn des Konstantstromimpulses und unmittelbar vor Abschalten des Konstantstromimpulses jeweils das Potential der Arbeitselektrode als erster, zweiter und dritter Potentialwert sowie die Länge des Konstantstromimpulses gemessen und gespeichert wird,

    der Serienwiderstand der Meßelektrode mittels Division der Potentialdifferenz aus dem zweiten und dem ersten Potentialwert durch den Wert des Konstantstromes ermittelt wird und

    die Phasengrenzkapazität der Arbeitselektrode mittels Quotientenbildung des Produktes aus Pulslänge und -wert des Konstantstromes und der Potentialdifferenz aus dem dritten und dem zweiten Potential ermittelt wird.

6.  Elektrostimulator nach Anspruch 5, **dadurch gekennzeichnet, daß**

    der Konstantstromimpuls aus einem Doppelpuls mit zueinander inverser Stromrichtung der beiden Teilpulse besteht und für jede Stromrichtung getrennt die Potentialdifferenzen, Pulslängen, Serienwiderstände und Phasengrenzkapazitäten der Meßelektrode ermittelt und anschließend hieraus die Mittelwerte für den Serienwiderstand und die Phasengrenzkapazität bestimmt werden.

7.  Elektrostimulator nach einem der Ansprüche 1 bis 6, **dadurch gekennzeichnet,**

    **daß** zur Bildung eines die Arbeitselektrode (102) enthaltenden Schwingkreises eine mit dem Ausgangsanschluß (101a) verbundene oder über ein Schaltelement (115) verbindbare Induktivität (116) vorgesehen ist,

    **daß** der Prüfsignalgenerator (114) einen, insbesondere abstimmbaren, Schwingungserzeuger aufweist und

die Auswertungseinrichtung (111) Mittel zur Bestimmung der Resonanzfrequenz des Schwingkreises bzw. eines Impedanzspektrogramms aufweist.

8. Elektrostimulator nach einem der vorhergehenden Ansprüche, **gekennzeichnet durch** die Ausführung als Herzschrittmacher (1; 101) zum Anschluß einer intrakardial angeordneten Herzschrittmacherelektrode (2; 102) als Arbeitselektrode.

9. Elektrostimulator nach Anspruch 8, **dadurch gekennzeichnet, daß** der Prüfsignalgenerator durch den Stimulationsimpulsgenerator (3) des Herzschrittmachers (1) gebildet ist, der zur Messung bevorzugt mit erniedrigter Impulsamplitude betrieben wird.

10. Elektrostimulator nach Anspruch 8 oder 9, **gekennzeichnet durch**

einen mit dem Ausgangsanschluß (1a; 101a) verbundenen Eingangsverstärker (4; 104) zur Verstärkung der über die Schrittmacherelektrode (2; 102) erfaßten elektrischen Herzsignale,

einen dem Eingangsverstärker (4; 104) nachgeschalteten Signaldetektor (6; 106) zur Detektion der Reizantwort des Herzens in dem Herzsignal und zur Erzeugung eines ersten Steuersignals in Reaktion auf das Ausbleiben der Reizantwort nach einem Stimulationsimpuls,

eine eingangsseitig mit dem Signaldetektor (6; 106) verbundene Steuerschaltung (7; 107), welche auf das Erscheinen des ersten Steuersignals hin einen ersten Steuervorgang bezüglich einer Schrittmacherfunktion oder eines Schrittmacherparameters und beim Ausbleiben des ersten Steuersignals einen zweiten diesbezüglichen Steuervorgang ausführt.

11. Elektrostimulator nach Anspruch 10, **dadurch gekennzeichnet, daß** die Auswertungseinrichtung (11; 111) ausgangsseitig zum Vergleich des die Elektrodenkapazität widerspiegelnden Ausgangssignals mit einem Vergleichswert mit einer Vergleichereinheit (12; 112) verbunden ist, welche beim Überschreiten des Vergleichswertes ein zweites Steuersignal erzeugt und daß die Steuerschaltung (7; 107) einen mit dem Ausgang der Vergleichereinheit (12; 112) verbundenen Steuereingang aufweist und den ersten oder zweiten Steuervorgang nur dann ausführt, wenn das zweite Steuersignal am Steuereingang anliegt.

12. Elektrostimulator nach Anspruch 10 oder 11, **dadurch gekennzeichnet, daß** die Steuerschaltung (7; 107) ausgangsseitig mit dem Stimulationsimpulsgenerator (3; 103) des Herzschrittmachers verbunden und derart ausgebildet ist, daß sie die Amplitude und/oder die Dauer der Stimulationsimpulse auf das Erscheinen des ersten Steuersignals hin erhöht und beim Ausbleiben des ersten Steuersignals verringert.

13. Elektrostimulator nach Anspruch 10 oder 11, **dadurch gekennzeichnet, daß** die Steuerschaltung (107) mit einem Steuereingang einer Einrichtung (117, 118) zur Anpassung der Stimulationsrate, die insbesondere zur Ratenanpassung in Reaktion auf ein intrakardial erfaßtes Signal ausgebildet ist, verbunden ist und in Abhängigkeit vom ersten und/oder zweiten Steuersignal eine Ratenadaption erlaubt oder inhibiert.

14. Elektrostimulator nach einem der Ansprüche 10 bis 143, **gekennzeichnet durch** einen der Vergleichereinheit (12; 112) zugeordneten, programmierbaren oder über eine Zugriffssteuerung mit dem Ausgang der Auswertungseinrichtung (111) verbundenen Vergleichswertspeicher (12a; 112a) zur Speicherung eines vorgegebenen oder bei vorangegangenen Auswertungen erhaltenen Vergleichswertes.

**Claims**

1. Electrical stimulator (1; 101) comprising an output terminal (1a; 101a) for an working electrode (2; 102) and a test signal generator (3; 114) connected on the output side to the output terminal for generating a pulsed or periodically changing test signal and for supplying the output terminal, a first measuring device (10; 110), connected in parallel with the test signal generator (3; 114) and connected on the input side to the output terminal, for measuring the electrical voltage on the output terminal, and an evaluating device (11; 111), connected on the input side at least indirectly to the first measuring device, for generating an output signal reflecting the capacitance of the working electrode as a function of current and/or voltage on the output terminal, **characterised in that** a current measuring device (8; 108) connected in series to the test signal generator and connected to the evaluating device (11; 111) is provided as the second measuring device for measuring the current flowing across the output terminal (1a; 101a) when a test signal is output.

2. Electrical stimulator according to claim 1, **characterised in that** an integrator (9) connected on the output side to the evaluating device (11) is connected downstream of the current measuring device (8) for determining the electrical load discharging during the pulse.

**3.** Electrical stimulator according to claim 1, **characterised in that** the test signal generator (3; 114) is configured for generating electrical pulses with an amplitude substantially below the amplitude of a stimulation pulse.

**4.** Electrical stimulator according to claim 1 or 3, **characterised in that** the first measuring device comprises a voltage measuring device (10; 110) connected in parallel with the test signal generator (3) and a galvanostatic control circuit is associated with the test signal generator (3) to keep the current flowing through the measuring electrode constant, the circuit comprising a power-supplying counter-electrode and a measuring current amplifier and a power amplifier.

**5.** Electrical stimulator according to claim 4, **characterised in that** the constant current flowing through the measuring electrode is pulsed and **in that** the respective potential of the working electrode is measured and stored as a first, second and third potential value and the length of the constant current pulse is measured and stored directly before and after the start of the constant current pulse and directly before switching off the constant current pulse, the series resistance of the measuring electrode is determined by dividing the potential difference of the second and the first potential value by the value of the constant current, and the phase limit capacitance of the working electrode is determined by forming the quotient of the product of the pulse length and value of the constant current and the potential difference of the third and the second potential.

**6.** Electrical stimulator according to claim 5, **characterised in that** the constant current pulse consists of a double pulse with mutually inverse current direction of the two partial pulses, and the potential differences, pulse lengths, series resistances and phase limit capacities of the measuring electrodes are determined separately for each current direction and the mean values for the series resistance and the phase limit capacitance are then determined herefrom.

**7.** Electrical stimulator according to any one of claims 1 to 6, **characterised in that** an inductance (116) connected to the output terminal (101a) or which can be connected via a switching element (115) is provided for forming an oscillating circuit containing the working electrode (102), **in that** the test signal generator (114) comprises an oscillation generator which, in particular, can be adjusted, and the evaluating device (111) comprises means for determining the resonance frequency of the oscillating circuit or of an impedance spectrogram.

**8.** Electrical stimulator according to any of the preceding claims, **characterised by** its construction as a cardiac pacemaker (1; 101) for connecting an intracardially arranged pacemaker electrode (2; 102) as the working electrode.

**9.** Electrical stimulator according to claim 8, **characterised in that** the test signal generator is formed by the stimulation pulse generator (3) of the pacemaker (1) which is preferably operated with a decreased pulse amplitude for measurement.

**10.** Electrical stimulator according to claim 8 or 9, **characterised by** an input amplifier (4; 104) connected to the output terminal (1a; 101a) for amplifying the electrical cardiac signals detected via the pacemaker electrode (2; 102), a signal detector (6; 106) connected downstream of the input amplifier (4; 104) for detecting the stimulation response of the heart in the cardiac signal and for generating a first control signal as a response to the non-appearance of the stimulation response after a stimulation pulse, a control circuit (7; 107) connected on the input side to the signal detector (6; 106) and which, on appearance of the first control signal, performs a first control operation with respect to a pacemaker function or a pacemaker parameter and, on non-appearance of the first control signal, performs a second control operation with respect thereto.

**11.** Electrical stimulator according to claim 10, **characterised in that** the evaluating device (11; 111) is connected on the output side to a comparator unit (12; 112) for comparing the output signal reflecting the electrode capacitance with a comparison value, the unit generating a second control signal when the comparison value is exceeded, and **in that** the control circuit (7; 107) comprises a control input connected to the output of the comparator unit (12; 112) and only performs the first or second control operation when the second control signal is present at the control input.

**12.** Electrical stimulator according to claim 10 or 11, **characterised in that** the control circuit (7; 107) is connected on the output side to the stimulation pulse generator (3; 103) of the pacemaker and is constructed in such a way that it increases the amplitude and/or the duration of the stimulation pulse on the appearance of the first control signal and reduces the first control signal on non-appearance.

**13.** Electrical stimulator according to claim 10 or 11, **characterised in that** the control circuit (107) is connected to a control input of a device (117, 118) for adjustment of the stimulation rate which is configured, in particular, for rate adjustment in response to an intracardially detected signal, and allows or inhibits a rate adaptation as a function of the first and/or second control signal.

14. Electrical stimulator according to any one of claims 10 to 13, **characterised by** a comparison value memory (12a; 112a) associated with the comparator unit (12; 112) and programmable or connected via an access controller to the output of the evaluating device (111), for storing a predetermined comparison value or a comparison value obtained during preceding evaluations.

## Revendications

1. Stimulateur électrique (1; 101) comportant:

    une borne de sortie (1a; 101a) pour une électrode de travail (2; 102) ainsi que
    un générateur de signal de contrôle (3; 114) relié côté sortie à la borne de sortie pour produire un signal de contrôle impulsionnel ou variable périodiquement et
    l'envoyer à la borne de sortie,
    un dispositif de mesure (10; 110) relié côté entrée à la borne de sortie et branché en parallèle avec le générateur de signal de contrôle (3; 114), pour la mesure de la tension électrique présente sur la borne de sortie,
    ainsi qu'un dispositif d'évaluation (11; 111) relié côté entrée au moins de façon indirecte au premier dispositif de mesure, pour produire un signal de sortie qui reproduit la capacité de l'électrode de travail, en fonction du courant et/ou de la tension sur la borne de sortie,

    **caractérisé en ce que**
    pour la mesure du courant, qui circule lors de la délivrance d'un signal de contrôle par l'intermédiaire de la borne de sortie (1a; 101a), en tant que second dispositif de mesure il est prévu un appareil de mesure de courant (8; 108) relié au dispositif d'évaluation (11; 111) et branché en série avec le générateur de signal de contrôle.

2. Stimulateur électrique selon la revendication 1, **caractérisé en ce qu'**un intégrateur (9) relié côté sortie au dispositif d'évaluation (11) est branché en aval de l'appareil de mesure de courant (8) pour déterminer la charge électrique s'évacuant lors de l'impulsion.

3. Stimulateur électrique selon la revendication 1, **caractérisé en ce que** le générateur de signal de contrôle (3; 114) est conçu de manière à produire des impulsions électriques possédant une amplitude qui est nettement inférieure à l'amplitude d'une impulsion de stimulation.

4. Stimulateur électrique selon la revendication 1 ou 3, **caractérisé en ce que**

    le premier dispositif de mesure comporte un appareil de mesure de tension (10; 110) branché en parallèle avec le générateur (3) de signal de contrôle, et
    au générateur de signal de contrôle (3) est associé, pour le maintien constant du courant circulant dans l'électrode de mesure, un circuit de régulation galvanostatique, qui comporte une contre-électrode d'amenée du courant ainsi qu'un amplificateur du courant de mesure et un amplificateur de puissance.

5. Stimulateur électrique selon la revendication 4, **caractérisé en ce que**

    le courant constant circulant dans l'électrode de mesure est de forme impulsionnelle, et
    juste avant et après le début de l'impulsion de courant constant et juste avant l'arrêt de l'impulsion de courant constant, respectivement le potentiel de l'électrode de travail en tant que première, seconde et troisième valeurs de potentiel ainsi que la longueur de l'impulsion de courant constant sont mesurées et mémorisées,
    la résistance série de l'électrode de mesure est déterminée au moyen de la division de la différence de potentiel entre les seconde et première valeurs de potentiel, par la valeur du courant constant, et
    la capacité de limite de phase de l'électrode de travail est déterminée au moyen de la formation du quotient du produit de la longueur d'impulsion par la valeur d'impulsion du courant constant, par la différence de potentiel entre les troisième et second potentiels.

6. Stimulateur électrique selon la revendication 5, **caractérisé en ce que**

    l'impulsion de courant constant est constituée par une impulsion double avec des sens réciproquement inverses du courant des deux impulsions partielles, et détermine, séparément pour chaque sens de courant, les différences de potentiel, les longueurs d'impulsions, la résistance série et les capacités de limite de phase de l'électrode de mesure et ensuite, à partir de là, les valeurs moyennes de la résistance série et de la capacité de limite de phase sont déterminées.

7. Stimulateur électrique selon l'une des revendications 1 à 6, **caractérisé en ce que** pour la formation d'un circuit oscillant contenant l'électrode de travail (102), il est prévu une inductance (116) reliée à la borne de sortie (101a) ou pouvant être reliée à cette borne de sortie par l'intermédiaire d'un élément de commutation (115),

que le générateur (114) du signal de contrôle possède un générateur d'oscillations notamment accordable, et

le dispositif d'évaluation (111) comporte des moyens pour déterminer la fréquence de résonance du circuit oscillant ou d'un spectrogramme d'impédance.

8. Stimulateur électrique selon l'une des revendications précédentes, **caractérisé par** la réalisation du stimulateur cardiaque (1; 101) pour le raccordement de l'électrode disposée de façon intracardiaque (2; 102) du stimulateur cardiaque, en tant qu'électrode de travail.

9. Stimulateur électrique selon la revendication 8, **caractérisé en ce que** le générateur de signal de contrôle est formé par le générateur (3) de l'impulsion de stimulation, qui fonctionne de préférence avec une amplitude réduite d'impulsion.

10. Stimulateur électrique selon la revendication 8 ou 9, **caractérisé par**

un amplificateur d'entrée (4; 104) relié à la borne de sortie (1a; 101a) pour amplifier les signaux cardiaques électriques détectés par l'intermédiaire de l'électrode (2; 102) du stimulateur;

un détecteur de signal (6; 106) branché en aval de l'amplificateur d'entrée (4; 104) pour la détection de la réponse d'excitation du coeur dans le signal cardiaque et pour la production d'un premier signal de commande en réaction à l'absence de la réponse d'excitation après une impulsion de stimulation,

un circuit de commande (7; 107) relié côté entrée au détecteur de signaux (6; 106) et qui exécute, lors de l'apparition du premier signal de commande, un premier processus de commande en rapport avec une fonction du stimulateur ou un paramètre du stimulateur et, dans le cas de l'absence du premier signal de commande, exécute un second processus de commande se rapportant à ce signal.

11. Stimulateur électrique selon la revendication 10, **caractérisé en ce que** le dispositif d'évaluation (11; 111) est relié côté sortie, pour la comparaison du signal de sortie reproduisant la capacité de l'électrode, à une valeur de comparaison, à une unité formant comparateur (12; 112), qui lors du dépassement de la valeur de comparaison, produit un second signal de commande et que le circuit de commande (7; 107) comporte une entrée de commande qui est reliée à la sortie de l'unité formant comparateur (12; 112) et exécute le premier ou le second processus de commande uniquement lorsque le second signal de commande est appliqué à l'entrée de commande.

12. Stimulateur électrique selon la revendication 10 ou 11, **caractérisé en ce que** le circuit de commande (7; 107) est relié côté sortie au générateur (3; 103) d'impulsions de stimulation du stimulateur cardiaque et est agencé de telle sorte qu'il augmente l'amplitude et/ou la durée des impulsions de stimulation lors de l'apparition du premier signal de commande et réduit cette amplitude et/ou cette durée dans le cas de l'absence du premier signal de commande.

13. Stimulateur électrique selon la revendication 10 ou 11, **caractérisé en ce que** le circuit de commande (107) est relié à une entrée de commande d'un dispositif (117, 118) servant à adapter la cadence de stimulation, qui notamment est conçu de manière à réaliser l'adaptation de cadence en réaction à un signal détecté de façon intracardiaque et autorise ou bloque une adaptation de cadence en fonction du premier et/ou du second signal de commande.

14. Stimulateur électrique selon les revendications 10 à 13, **caractérisé par** une mémoire programmable de valeur de comparaison (12a; 112a), associée à l'unité formant comparateur (12; 112) ou reliée par l'intermédiaire d'une commande d'accès à la sortie du dispositif d'évaluation (111) et servant à mémoriser une valeur de comparaison prédéterminée ou obtenue lors d'évaluations précédentes.

Fig.1

$U_{Stim}$

$C_H$

$R_L$

$C_{min}$

$C_H$

MEM

COMP

ALU

$\int idt$

U

Q

i

$R_F$

EP 0 913 166 B1

Fig.2

Fig.3a

Fig.3b

Fig.4